# EUROPEAN PATENT APPLICATION

(11) **EP 1 090 646 A2**
(43) Date of publication of application: **11.04.2001**
(21) Application number: 00870218.5
(22) Date of filing: 27.09.2000
(51) Int. Cl.: A61K 51/04

(54) **Labelled d-mannoheptulose derivatives and their use for imaging of the endocrine pancreas**

(30) Priority: 30.09.1999 EP 99870201
(71) Applicant: UNIVERSITE LIBRE DE BRUXELLES, 1050 Bruxelles (BE)
(72) Inventor: Malaisse, Willy, 1180 - Bruxelles (BE)
(74) Representative: Van Malderen, Joelle

(57) **Abstract**

The present invention is related to labelled D-mannoheptulose or labelled D-mannoheptulose derivatives used for imaging endocrine pancreas tissues or cells.

## Description

### Field of the invention

The present invention is related to a labelled D-mannoheptulose and labelled D-mannoheptulose derivatives and to a kit comprising them.

The present invention is also related to the use of these molecules and this kit for improving imaging of the endocrine pancreas by a non-invasive method.

### Background of the invention

The imaging of the endocrine pancreas (islets of Langerhans) by a non-invasive method would represent a major breakthrough for the characterisation of the mass of insulin-producing cells and choice of treatment in patients with non-insulindependant (type 2) diabetes mellitus.

Thus, D-mannoheptulose, which is a specific inhibitor of D-glucose phosphorylation to D-glucose 6-phosphate by hexokinase isoenzymes, inhibits D-glucose metabolism in isolated pancreatic islets and hepatocytes, namely the two sole cell types expressing the GLUT2 type of carriers for monosaccharide transport across the plasma membrane (A. Sener, et al., Diabetologia 41, pp. 1105-1113 (1998); L. Ladrière, et al., Int. J. Mol. Med. 1, pp. 967-970 (1998)).

Other cell types, such as erythrocytes and parotid cells are resistant to the inhibitory action of D-mannoheptulose upon D-glucose metabolism (L. Ladrière, et al., Horm. Metab. Res. 30, pp. 244-245 (1998); W. J. Malaisse, et al., Biochem Mol. Biol. Internat. 44, pp. 625-633 (1998)). Yet, D-mannoheptulose is perfectly able to inhibit D-glucose phosphorylation in homogenates of either erythrocytes or parotid cells (C. Vanhoutte and W.J. Malaisse, Med. Sci. Res. 25, pp. 735-737 (1997), O. Scruel, et al., Mol. Coll. Biochem. 187, pp. 113-120 (1998)). Hence, the failure of D-mannoheptulose to inhibit D-glucose metabolism in intact erythrocytes and parotid cells is likely to be attributable to the fact that the heptose would not be transported into these cells. A further argument in support of the latter proposal was found in the following experiments. At variance with unesterified monosaccharides, the polyacetate ester of several monosaccharides enter into cells without requiring the intervention of a specific carrier system; they are then hydrolysed inside the cells in esterase-catalysed reactions, so that the unesterified monosaccharide becomes eventually available to undergo intracellular metabolism or to exert intracellular effects (W. J. Malaisse, et al., Biochem. Biophys. Res. Commun. 231, pp. 435-436 (1997)). Advantage was taken of such a situation to demonstrate that D-mannoheptulose hexaacetate inhibits D-glucose metabolism in intact erythrocytes and parotid cells, in sharp contrast to the results obtained with unesterified D-mannoheptulose (L. Ladrière, et al., Horm. Metab. Res. 30, pp. 244-245 (1998); W. J. Malaisse, et al., Biochem. Mol. Biol. Internat. 44, pp. 625-633 (1998)).

### Aims of the invention

The aim of the present invention, is to provide labelled compounds, preferably radioactive compounds, suitable for improving imagining of the endocrine pancreas by a non-invasive method, especially compounds which could be used easily for quantification of endocrine pancreatic mass and could thereafter be used for improving the diagnostic of type I or type II diabetes or cancer.

### Summary of the invention

The present invention is related to a labelled D-mannoheptulose and labelled D-mannoheptulose derivatives, preferably a radioactive D-mannoheptulose and radioactive D-mannoheptulose derivatives as well as to a kit comprising them. Such kit comprises said elements for imaging the endocrine pancreas.

Preferably, the above-mentioned molecules are radioactive molecules that are advantageously labelled with either ¹¹C, ¹⁸ F or radioactive iodine (¹²³I and/or ¹³¹I).

However, other labelled molecules specifically selected by the person skilled in the art could be used for said in vitro and/or the in vivo analysis.

The inventors have found unexpectedly, that these compounds are suitable for obtaining the specific labelling (and imaging upon a screen) of the endocrine moiety of the pancreatic tissues, (in vitro and in vivo) after injection of this labelled molecules to a mammal.

Said imaging, is particularly suitable for the identification of endocrine pancreatic tissues but also for the quantification of endocrine pancreatic mass, which is extremely useful for the diagnostic and or monitoring of specific diseases affecting said endocrine pancreatic tissues, such as type I and type II diabetes or cancer.

Therefore, another aspect of the present invention is related to the use of said labelled molecules in the imaging of endocrine pancreas tissues of a patient.

A specific aspect of the present invention is related to a ("non-invasive") method for imaging endocrine pancreas tissues or cells of a patient, comprising the step of administrating to said patient of a suitable amount of the labelled molecules according to the invention, detecting and possibly recording a signal obtained from said labelled molecules according to the invention in the endocrine pancreas tissues or cells of said patient by imaging detecting (and recording) means. Such imaging method could be applied in vitro (by recovering cells from a patient) upon tissues or cells, or in vivo (upon the patient). Preferably, the detecting and recording means are well-known by persons skilled in the art, such as means used in positron-emission tomography, resulting from a radioactive emission by said radioactive label.

Preferably, such method further comprises the step of correlating the signal obtained to a corresponding reference signal (a reference signal obtained from a labelled D-mannoheptulose or a labelled D-mannoheptulose derivative, administrated to a healthy patient (preferably an adult patient) and present in the endocrine pancreas tissue or cell from said healthy patient, and possibly the diagnostic of a disease affecting the cells or a tissue of said tested patient.

The present invention is related also to a computer program comprising program code means for performing the step according to the invention, once said program is stored on computer and related to a computer program further comprising program code means stored on computer readable means for performing the steps of the method according to the invention when said program is stored on the computer.

The D-mannoheptulose, could be extracted and purified from avocados and from pentose or hexose precursors. Preferably, the labelled D-mannoheptulose or its derivatives are identified in imaging by position emission tomography in a manner comparable to the currently used to study D-glucose transport in other organs, for instance in human brain by using 3-O- [¹¹C] methyl-D-glucose (L.E. Feinendagen, et al., J. Nucl. Med. 27, pp. 1867 - 1879 (1986)).

As the time required for the synthesis of several compounds, especially ¹¹C-labelled D-mannoheptulose may represent several drawbacks, taking into account the short half-life of ¹¹C, an alternative approach consist in the use of 3-deoxy-3-[¹⁸F] fluoro-D-mannoheptulose in a manner comparable to the use of 2-deoxy-2-[¹⁸F] fluoro-D-glucose, the most frequently used radiopharmaceutical for position emission tomography.

The approach for the synthesis of the ¹⁸F-labelled D-mannoheptulose derivative could be adapted from that currently used for the synthesis of 2-deoxy-2-[¹⁸F] fluoro-D-glucose (C. Lemaire, et al., J. Labelled Comp. Radiopharm. 40, pp. 256-257 (1997) and WO97/42203). In considering this approach, it should be kept in mind that, in the use of 2-deoxy-2-[¹⁸F] fluoro-D-glucose, advantage is taken of its phosphorylation to its 6-phosphate ester by hexokinase to allow the intracellular accumulation of this ester, which is not further metabolised. Observations conducted by the inventors suggest that D-mannoheptulose undergoes phosphorylation by hexokinase, as judged from the rate of conversion of ATP to ADP or from the conversion of D-[H³] mannoheptulose to its acidic metabolite.

A preferred and probably most useful tool for the imaging of the endocrine pancreas, proposed in this invention, consists of iodinated D-mannoheptulose derivatives labelled with ¹³¹I or ¹²³I, the uptake of which by insulin-producing cells could be measured by single photon emission computed tomography. The pilot molecule proposed in this respect would be 7-deoxy-7-iodo-D-mannoheptulose. Indeed, by analogy of works made by C. Henry et al., Nucl. Med. Biol. 24, pp. 527-534 (1997) with 6-deoxy-6-iodo-D-glucose labelled with ¹²³I or ¹²⁵I, iodinated D-mannoheptulose derivatives display the same cell-specificity, in terms of uptake by insulin-producing cells *versus* acinar pancreatic cells, as D-mannoheptulose itself. Indeed, the recent work made with 6-deoxy-6-iodo-D-glucose has demonstrated that this D-glucose derivative is a reliable tool to trace D-glucose transport in several distinct cell types.

The present invention is not restricted to labelled (radioactive) 7-deoxy-7-iodo-D-mannoheptulose and covers other labelled, preferably iodinated derivatives of D-mannoheptulose, such as 1-deoxy-1-iodo-D-mannoheptulose and the iodoethoxy and iodophenyl analogues of D-mannoheptulose.

The present invention will be described in detail in the following non-limiting examples in reference to the following figures, presented as preferred illustrations of the various embodiments of the present invention.

### Brief description of the drawings

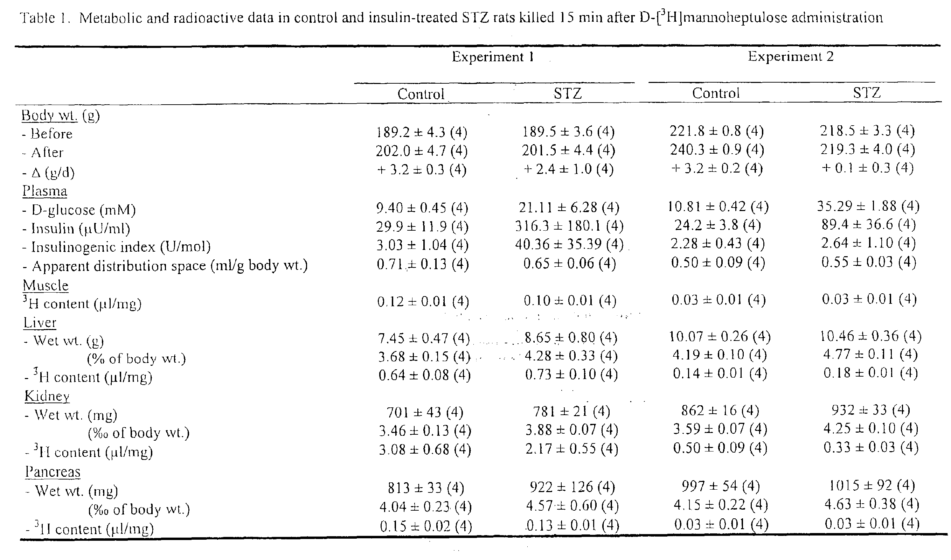

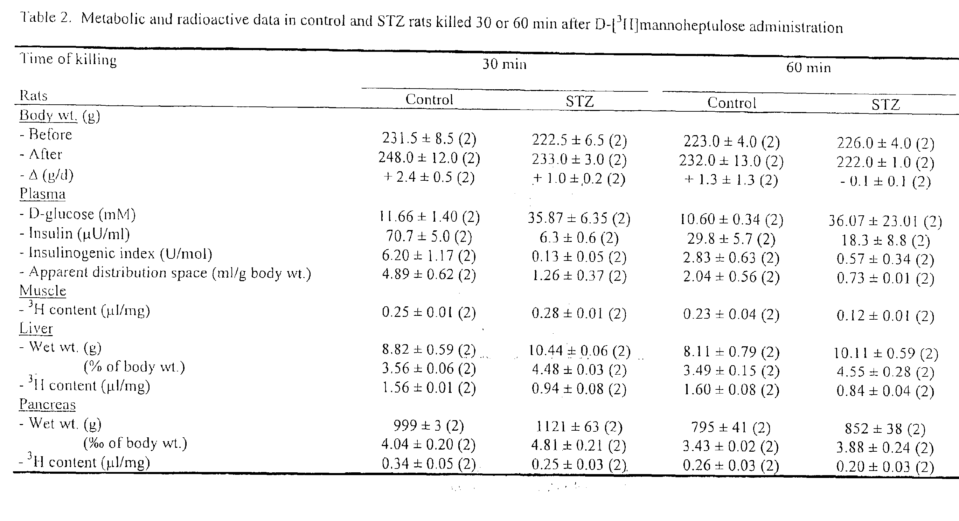

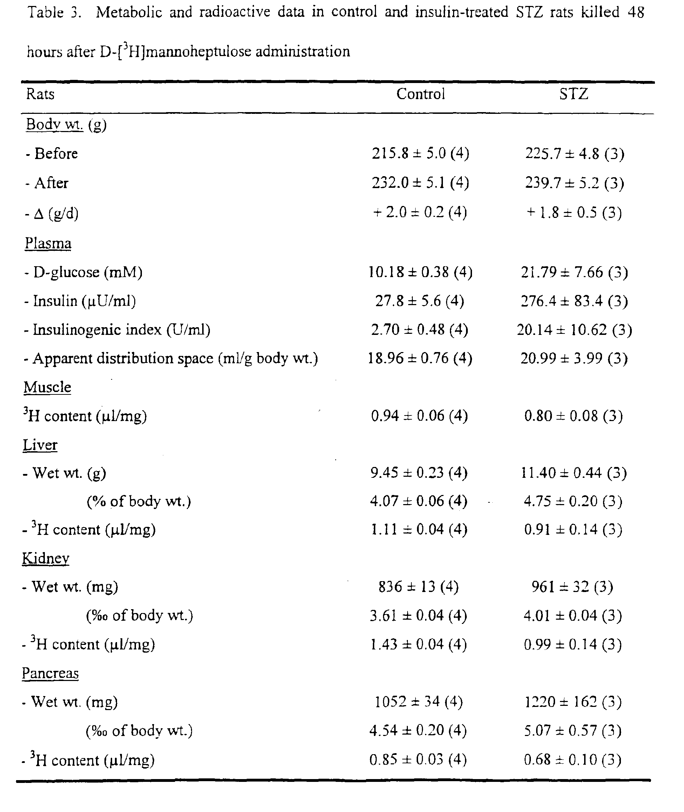

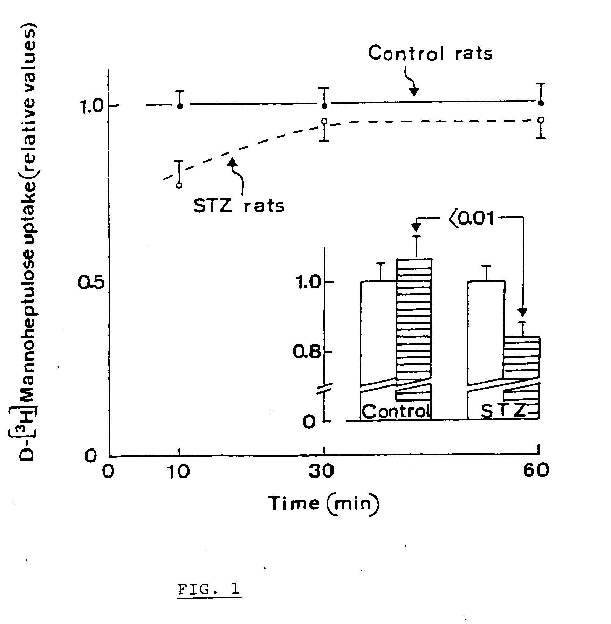

Figure 1 represents time course of D-[³H]mannoheptulose (0.1 mM) apparent distribution space in pieces of pancreas from control (closed circles and solid line) and STZ rats (open circles and dashed line) incubated in the presence of 8.3 mM D-glucose. Mean values ± SEM (n = 22-24) are expressed relative to the corresponding control values found at the same time within the same experiment(s). The inset illustrates the effect of D-glucose (8.3 mM, shaded columns), at the 60^{th} min of incubation, upon the apparent distribution space of D-[³H]mannoheptulose (0.1 mM) by pieces of control and STZ rats, relative to the corresponding reference value found in the same type of rats and within the same experiments in the absence of the hexose (open columns); mean values refer to 22-24 individual determinations.

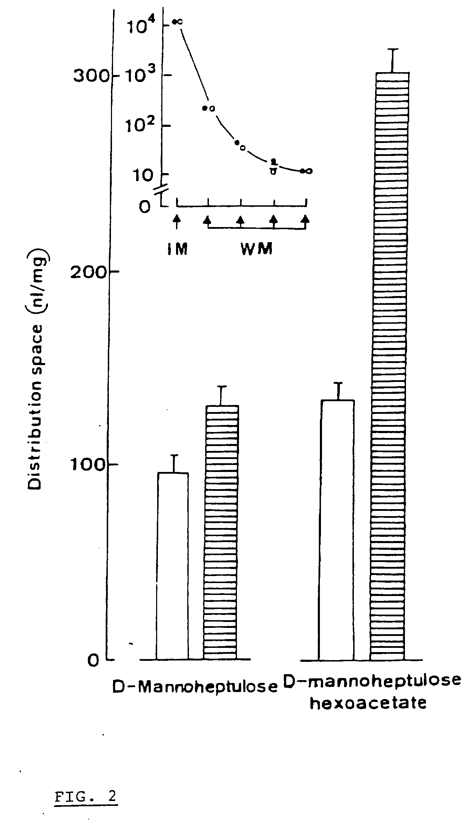

Figure 2 represents parent distribution space (nl/mg wet wt.) of D-[³H]mannoheptulose (0.1 mM; left) and D-[³H]mannoheptulose hexaacetate (0.02 mM; right) in pieces of pancreas from normal rats incubated for either 10 min (open columns) or 60 min (hatched columns) at 37°C in the presence of 8.3 mM D-glucose; mean values (± SEM) refer to 8 individual observations in all cases. The inset illustrates in semi-logarithmic co-ordinates the progressive decrease in the radioactive content of the incubation medium (IM) and washing media (WM) during the washing procedure of pieces first incubated in the presence of either D-[³H]mannoheptulose (closed circles) or its hexaacetate ester (open circles); mean values (± SEM) refer to 8-16 individual observations and are also expressed as an apparent distribution space (nl/mg wet wt.).

### Example 1: D-[1-¹¹C] Mannoheptulose synthesis

0.1 mmoles Formaldehyde/Paraformaldehyde solution was taken to dryness and was added to 0.1 mmoles (6.1 mg, 5.4 µl) Nitromethoane, stirred at room temperature and added to 0.01 mmoles (3.5 µl) of 3N potassium hydroxide solution in methanol, stirred for 5 minutes at room temperature.

Therefore, said solution was added to 0.09 mmoles (31.5 µl) of 3N potassium hydroxide solution in methanol, stirred 10 minutes at room temperature and was added to a slurry of 0.1 mmole 5% D-Arabinose in water/methanol (50:50) [15 mg D-Arabinose in 150 µl water/150 µl methanol] . The arabinose will go into a solution as the nitroethanol is added. The solution is stirred 15 minutes at room temperature and upon said solution is added 0.2 mmoles of 20% aqueous sulphuric acid (iced cold) [10.5 µl H₂SO₄/40 µl ice cold water]. The solution was allowed to warm to room temperature and stirred 15 minutes, before being neutralised with calcium carbonate and filtered, deionized and then purified on an Aminex type column.

The recovered molecule is a radioactive molecule of D-[1-¹¹C] mannoheptulose. Such compound, could be used like D-[³H] or D-[1-¹⁴C]-mannoheptulose molecules described in the following examples.

### Example 2: D-[³H] Mannoheptulose Distribution in Streptozotocin-Induced Diabetic Rats Transplanted with Islets in an Implantation Module

Female Wistar rats (220-230 g body wt.) were injected intravenously with streptozotocin (0.25 µmol/g body wt.) on day zero. Eight to nine days later, they were transplanted with 1,500-6,000 islets prepared by the collagenase procedure (2) from rats of the same sex and strain (Iffa Credo, L'Arbresle, France). The islets were placed (1,500 islets per module) in 1-4 implantation modules (Statice Santé, Besançon, France), which were inserted, under pentobarbital anaesthesia (0.5-0.6 µmol/g body wt.; Nembutal, Sanofi, Brussels, Belgium), into the peritoneal cavity. The modules were equipped with a 20 µm thick polycarbonate membrane (Whatman, Maidstone, Kent, UK) with 6 x 10⁸ pores (diameter : 50 nm) per cm². The glycemia of the rats was monitored in blood samples collected from the severed end of the tail.

D-[³H]mannoheptulose (100 µCi in 0.3 ml saline containing 0.1 mM unlabelled D-mannoheptulose) was injected intraperitoneally and the animals killed by decapitation 24 hours later after a last blood sampling from the severed end of the tail had been obtained for measurement of glycemia. The blood collected after decapitation in heparinized vials was used for measurements of radioactivity in aliquots of blood, plasma and erythrocytes, as well as for determination of plasma D-glucose and insulin concentration. Samples of the liver, gastrocnemius muscle and kidney, as well as the whole pancreas, were homogenised in 1.5 ml H₂O, aliquot parts of the homogenates being used for DNA and radioactive measurements. The fluid present in the implantation device was aspirated and also examined for its radioactive content. Thereafter, the implantation device was repeatedly frozen and thawn in 0.4 ml H₂O, aliquot parts of this extract being then again used for both DNA and radioactive determinations.

The methods used to measure DNA content (3), glycemia (Glycotronic C; Macherey-Nagel, Düren, Germany), plasma D-glucose (4) and insulin (5) concentrations were previously described in the cited references.

D-[³H]mannoheptulose was injected intraperitoneally in streptozotocin-induced diabetic rats, that had been transplanted with islets placed in an implantation module. One day after the administration of the tritiated heptose, its volume of distribution, as judged from the plasma radioactive content, averaged 10.0 ± 2.2 ml/g body weight. The radioactive content (dpm/mg wet weight) of the liver (64.5 ± 6.4), kidney (51.0 ± 0.9), muscle (28.9 ± 7.8) and pancreas (28.7 ± 2.6), when compared to that calculated for the transplanted islets (332.7 ± 30.7) yielded a paired islet/pancreas ratio of 11.59 ± 0.03.

In these mice, the radioactive content of the transplanted islets was, 60 min after the intravenous injection of D-[³H]mannoheptulose, also about 10 times higher than that found in the pancreas. In this prior study as in the present experiments, the pancreatic radioactive content is assumed to reflect the situation prevailing in acinar cells, since insulin-producing cells were previously suppressed as a result of streptozotocin administration.

The calculated radioactive content of the transplanted islets (dpm/mg wet wt.) was 6.49 ± 1.27 higher than the paired plasma radioactive content (dpm/µl), a favourable situation in the perspective of the imaging of the endocrine pancreas.

These converging observations thus support the view that radioactive D-mannoheptulose could be used as a tool to label preferentially the endocrine moiety of the pancreatic gland.

However, since the islet B-cells account for no more than about 1.0 percent of the total pancreatic mass, even a tenfold difference in the uptake of radioactive D-mannoheptulose (or a radiolabelled analog of the heptose suitable for non-invasive imaging by positron emission tomography or single photon emission computerised tomography) would only result in a modest difference of about 10 % when comparing the total radioactive content of the pancreatic gland in control and type-1 diabetic animals or human subjects.

### Example 3: Pancreatic Fate of D-[³H] Mannoheptulose

Two preparations of D-[³H]mannoheptulose were used. The first preparation, used in a few *in vitro* experiments and in one set of *in vivo* experiments (animals injected 30 to 60 min before sacrifice), was a product from Nycomed Amersham (Little Chalfont, Buckinghamshire, UK). Its radiochemical purity, as assessed by thin layer chromatography on cellulose, ranged from 89 to 98 %, depending on the elution system used for such a purpose. The second preparation used in all other experiments was a product from NEN Life Science Products (Zaventem, Belgium). Its specific radioactivity was 37 Ci/mmol and radiochemical purity above 95 %, as determined by HPLC. In all experiments conducted *in vivo,* the animals were injected intravenously with D-[³H]mannoheptulose (10-40 µCi, and 200 µCi in the case of the rats killed 48 hours after this injection) placed in 300 µl of saline. Except in the animals killed 30-60 min after D-[³H]mannoheptulose injection, the inoculate also contained 0.1 mM unlabelled D-mannoheptulose.

The hexaacetate ester of unlabelled or tritiated D-mannoheptulose was synthesised by a method adapted from that described elsewhere (Vogel, A.I. (1956). *A Textbook of Practical Organic Chemistry,* 3^{rd} edn. Langmans Green: London, pp. 451-452).

The method used to prepare pancreatic pieces and isolated pancreatic islets¹¹ and for measuring the apparent distribution space of ³HOH and D-[³H]mannoheptulose was comparable to that described elsewhere (Ladrière, L., Malaisse-Lagae, F. and Malaisse, W.J. (2000). Pancreatic uptake of ⁶⁵Zn in control and streptozotocin-injected rats. *Med. Sci. Res.,* **28**, 43-44).

Unless otherwise mentioned, the pancreatic pieces or isolated islets were submitted after incubation, to four successive washes conducted at 4°C with a medium containing 20.0 µM cytochalasin B (Sigma, St. Louis, MO, USA). In these experiments, control rats were compared to diabetic animals, that had been injected intravenously with streptozotocin (Sigma), at a dose of 0.25 µmol/g body wt., 4-38 days before sacrifice. Over this period, the STZ rats lost 0.8 ± 0.3 g/d (*n* = 6), whilst the control rats gained 1.1 ± 0.2 g/d (*n* = 5; *P* < 0.001). The final body wt. averaged 254.2 ± 18.4 g (*n* = 5) and 220.0 ± 14.3 g (*n* = 6) in control and STZ rats, respectively. The plasma D-glucose¹³ and insulin¹⁴ concentrations averaged, respectively, 37.76 ± 3.28 mM and 4.6 ± 1.4 µU/ml in the STZ rats (*n* = 6), as distinct (*P* < 0.05 or less) from 7.30 ± 1.53 mM and 32.7 ± 13.4 µU/ml in control rats (*n* = 4-5).

In the *in vivo* experiments, the STZ rats were either not treated with insulin (animals killed 30-60 min after D-[³H]mannoheptulose injection) or received twice daily 6 U of insulin (Insulatard, Novo Nordisk, Bagsvaerd, Denmark) subcutaneously, the last of these injections being given at about 5 p.m. on the day before either the injection of D-[³H]mannoheptulose (animals killed 15 min after D-[³H]mannoheptulose administration) or the sacrifice of the rats (rats examined 48 hours after D-[³H]mannoheptulose injection). The animals killed 15 min after the injection of D-[³H]mannoheptulose received a total of either 5 insulin injections, the first being made at about 5 p.m. on the day after streptozotocin administration, or 6 insulin injections given between the third and fifth day after streptozotocin administration. In the rats killed 48 hours after the injection of D-[³H]mannoheptulose, the insulin treatment was initiated at 9 a.m. 3 days after streptozotocin administration and continued for a total of 5 days.

At stated times after D-[³H]mannoheptulose intravenous injection, the rats were killed by decapitation, blood was collected in heparinized vials and the plasma separated for measurement of its radioactive content, as well as D-glucose¹³ and insulin¹⁴ concentrations. Samples of gastrocnemius muscle, liver, kidney and pancreas were weighed, homogenised in 1.5-3.0 ml of water, and aliquot parts of these homogenates examined for their radioactive content by liquid scintillation.

All radioactive data were calculated as dpm/µl of plasma or dpm/mg wet wt. (muscle, liver, kidney, pancreas). In these four tissues, the results were eventually expressed as µl/mg wet wt., by dividing the radioactive content (dpm/mg) by the paired plasma value (dpm/µl).

All results are presented as mean values (± SEM, or individual deviation from mean value whenever *n* = 2) together with the number of individual measurements (*n*) or degree of freedom (*d.f.*). The statistical significance was assessed by use of Student's *t*-test.

In pilot experiments conducted in isolated islets submitted to only 3 washes at 4°C with a medium containing cytochalasin B (20.0 µM), the apparent distribution space of D-[³H]mannoheptulose (0.1 mM) after 10-120 min incubation at 37°C, amounted to 5.5 nl/islet or more, indicating incomplete removal of interstitial radioactive material. In these experiments, no significant difference was found between the two commercial preparations of tritiated D-mannoheptulose, whether after 10 or 30 min incubation at 37°C. The apparent distribution space of D-[³H]mannoheptulose was higher (*P* < 0.02), however, after incubation for 30 or 120 min at 37°C in the presence of D-glucose (8.3 mM) rather than in its absence, the former value averaging 161.4 ± 23.7 % (*n* = 14) of the mean corresponding reference value (no D-glucose) found within the same experiments after the same length of incubation (100.0 ± 8.3 %; *n* = 16).

In a further series of experiments, groups of 20 islets each were submitted, after incubation, to 6 successive washes at 4°C in the presence of cytochalasin B (20.0 µM). Under these conditions, it was possible to document that the apparent distribution space of D-[³H]mannoheptulose (0.1 mM) (i) increased by 73.5 ± 30.3 % (*d.f. =* 8; *P* < 0.05) between the 15^{th} and 120^{th} min of incubation at 37°C in the presence of 8.3 mM D-glucose, (ii) decreased, after 30 min incubation at 4°C in the absence of D-glucose, virtually to the same low value (*P* > 0.6; *d.f. =* 14) as that found after incubation in the presence of D-[5-³H]glucose (8.3 mM) for the same time and at the same temperature, the latter value representing only 55.5 ± 8.3 % (*n* = 8; *P* < 0.05) of that found after incubation in the presence of the tritiated hexose (8.3 mM) for 30 min at 37°C, and (iii) represented, after 30 min incubation at 37°C in the absence of D-glucose, only 77.3 ± 9.4 % (*n* = 6) of the mean value found within the same experiments after incubation in the presence of D-[5-³H]glucose (8.3 mM) for the same time and at the same temperature.

In the last set of experiments in this series, groups of 20 islets each were incubated for 60 min at 37°C in the presence of D-[³H]mannoheptulose (0.1 mM), washed thrice at 4°C, further incubated for 60 min at 37°C in the absence of the heptose but presence of cytochalasin B (20.0 µM), again submitted to 4 successive washes, and eventually examined for their radioactive content. Under these experimental conditions, the radioactive content of islets first incubated in the presence of 8.3 mM D-glucose again exceeded (*P* < 0.02) that found in islets first incubated in the absence of the hexose, the values recorded in the former case averaging 168.5 ± 19.1 % (*n* = 8) of those found in the latter situation (100.0 ± 11.4 %; *n* = 6). In these experiments, the incorporation of cytochalasin B (20.0 µM) in the first incubation medium containing 8.3 mM D-glucose decreased (*P* < 0.05) the final radioactive content to 103.7 ± 14.7 % (*n* = 6) of the reference value after the first incubation in the sole presence of D-[³H]mannoheptulose.

Over 10 to 60 min incubation at 37°C in the presence of 8.3 mM D-glucose, the mean apparent distribution space of D-[³H]mannoheptulose (0.1 mM) in pieces of pancreas from STZ rats was always somewhat lower than in control rats, the overall mean value in the diabetic animals representing 89.2 ± 3.6 % (*n* = 72; *P* < 0.02) of the corresponding mean control value found at the same time within the same experiment(s) (100.0 ± 2.5 %; *n* = 66). As illustrated in Figure 1, the difference between STZ and control rats was quite obvious after 10 min incubation (*P* < 0.01), but faded out after 30-60 min incubation. Another difference between STZ and control rats consisted in the fact that, over 60 min incubation, the apparent distribution space of D-[³H]mannoheptulose was significantly lower (*P* < 0.02) in the presence of D-glucose (8.3 mM) than in its absence in the diabetic animals, but not so in the control rats (Figure 1, inset). Thus, relative to the mean value recorded within the same experiment(s) in the absence of D-glucose, the apparent distribution space of D-[³H]mannoheptulose found in the presence of the hexose averaged 83.7 ± 4.5 % (*n* = 24) in the STZ rats, as distinct (*P* < 0.01) from 106.7 ± 6.5 % (*n* = 22) in the control animals. In both control and STZ rats, however, the apparent distribution space of D-[³H]mannoheptulose (0.1 mM) over 60 min incubation in the presence of 8.3 mM D-glucose failed to be significantly affected by the presence of cytochalasin B (20.0 µM) in the incubation medium, the experimental readings averaged 101.1 ± 4.1 % (*n* = 48; pooled data obtained in control and STZ rats) of the mean reference value (no cytochalasin B) found within the same experiment(s) in the same type of rats (100.0 ± 3.4 %; *n* = 46).

In the next series of experiments, the possible uptake of D-[³H]mannoheptulose was compared to that of its hexaacetate ester in pieces of pancreas from normal rats (Figure 2). After both 10 and 60 min incubation, the apparent distribution space of D-[³H]mannoheptulose hexaacetate (0.02 mM) exceeded (*P* < 0.025 or less) that of the unesterified heptose (0.1 mM). Moreover, the ratio between the mean values recorded after 60/10 min incubation was much higher (*P* < 0.005) in the case of D-[³H]mannoheptulose hexaacetate (2.26 ± 0.17) than in the case of the unesterified heptose (1.36 ± 0.16). Figure 2 also illustrates the progressive decrease in the radioactive content of the incubation and washing media during the washing of the pieces of pancreas first exposed to D-[³H]mannoheptulose or its hexaacetate ester.

In the last set of experiments in this series, pieces of pancreas from either normal or STZ rats were first incubated for 60 min at 37°C in the presence of 8.3 mM D-glucose, then submitted to 3 washes at 4°C in the presence of the hexose (8.3 mM) and cytochalasin B (20.0 µM), further incubated for 60 min at 37°C, still in the presence of D-glucose and the mould metabolite, and eventually submitted to 4 washes at 4°C prior to measurement of their radioactive content. Under these experimental conditions, the ³HOH apparent distribution space represented no more than 1.8 ± 0.4 % (*n* = 16) of that measured, within the same experiments, when the pieces of pancreas were first incubated for 60 min at 37°C and then immediately submitted to 4 washes at 4°C before measuring their radioactive content. In the case of D-[³H]mannoheptulose (0.1 mM), the corresponding value averaged 14.4 ± 0.4 % (*n* = 8) in pancreatic pieces from control rats and 9.4 ± 0.4 % (*n* = 8) in those from STZ rats. This procedure also allowed to document that, in both control and STZ rats, the final distribution space of D-[³H]mannoheptulose after the two successive incubations largely exceeded that of ³HOH (*P* < 0.001). It further revealed that such a final distribution space of D-[³H]mannoheptulose, relative to that of the initial incubation medium radioactive content (also expressed as nl/mg wet wt.) was higher (*P* < 0.02) in control animals (1.78 ± 0.10 %; *n* = 8) than in STZ rats (1.34 ± 0.11 %; *n* = 8). Last, it indicated that the final distribution space of the tritiated heptose was much lower when the first incubation was conducted at 4°C, instead of 37°C, the former value averaging, in control and STZ rats respectively, 36.9 ± 4.2 and 38.0 ± 3.9 % of the latter one (100.0 ± 5.3 and 100.0 ± 3.3 %; *n* = 8 in all cases).

Whilst the control rats gained weight at a mean rate of 2.6 ± 0.2 g/d (*n* = 16), the rats injected with STZ lost 3.4 ± 1.1g/d (*n* = 11) after intravenous injection of the β-cytotoxic agent and then gained 5.7 ± 0.8 g/d (*n* = 7) when treated with insulin. In the latter situation, 8 animals remained markedly hyperglycaemic and glycosuric, whilst the other 3 rats were non glycosuric with a mean plasma D-glucose concentration of 10.00 ± 3.21 mM. In all cases, the last subcutaneous injection of insulin was administered at about 5 p.m. on the day preceding the experiment. The concentration of insulin was increased (*P* < 0.01) in the insulin-treated STZ rats, averaging 222.9 ± 71.5 µU/ml (*n =* 11) as compared to 27.3 ± 4.2 µU/ml (*n =* 12) in the corresponding control rats. Likewise, the mean insulinogenic index (i.e. the paired ratio between plasma insulin/D-glucose concentration) was higher, albeit not significantly so, in insulin-treated STZ rats (21.13 ± 12.79 U/mol; *n* = 11) than in control animals (2.67 ± 0.38 U/mol; *n* = 12). When the STZ rats were not treated with insulin, both their plasma insulin concentration (12.3 ± 5.0 µU/ml) and insulinogenic index (0.35 ± 0.19 U/mol) were much lower (*P* < 0.02 or less) than in the corresponding control animals (59.1 ± 8.5 µU/ml and 4.52 ± 1.12 U/mol; *n* = 4 in all cases). The wet wt. of the liver and kidney was also higher (*P* < 0.02 or less) in insulin-treated STZ rats than in the corresponding control animals. When expressed relative to the mean value found in the corresponding control rats, the absolute value for the wet wt. of liver and kidney averaged, in the insulin-treated STZ rats, respectively 112.9 ± 4.5 and 111.0 ± 2.0 % (*n* = 11 in both cases). When expressed relative to the paired body wt., such percentages amounted to 115.5 ± 3.3 % for the liver and 114.1 ± 1.6 % for the kidney (*n* = 11 in both cases). The pancreas wet wt. was not significantly different in insulin-treated STZ rats and control animals, whether expressed in absolute terms or relative to paired body wt. In the STZ rats not treated with insulin, the liver wet wt. was also higher (*P* < 0.02 or less) than in the corresponding control animals, whether expressed in absolute terms or relative to paired body wt.

The radioactive content of the pancreatic gland, relative to the paired plasma value, was higher in normal rats than in diabetic animals killed 15-2880 min after intravenous injection of D-[³H]mannoheptulose (Tables 1 to 3). Pooling all available data, the results recorded in STZ rats indeed averaged 81.6 ± 4.2 % (*n* = 15; *P* < 0.02) of the mean control value found within the same experiments and at the same time after injection of D-[³H]mannoheptulose in normal animals (100.0 ± 6.0 %; *n* = 16). As already observed in a prior study,¹⁵ the results obtained shortly (15 min) after injection of the tritiated heptose were less well grouped than those recorded at later times. When the former results were not taken into account, the mean value in STZ rats averaged 77.9 ± 5.6 % (*n* = 7; *P* < 0.006) of the corresponding mean control value (100.0 ± 3.8 %; *n* = 8).

The situation found in the pancreatic gland differed from that found in the liver, in which case the paired ratio between liver/plasma radioactivity represented in the STZ rats 109.5 ± 8.3 % (*n* = 11) of that recorded, within the same experiments and at the same time, in the control rats (100.0 ± 4.3 %; *n* = 12; *P* > 0.3). A low liver/plasma radioactive ratio (56.1 ± 3.3 % of the mean corresponding value found in normal rats; *n* = 4) was only observed in the few STZ animals that were not treated with insulin and injected with only a tracer amount of tritiated D-mannoheptulose in the absence of the unlabelled heptose.

Likewise, the paired ratio between muscle/plasma radioactivity was not significantly different in STZ rats and control animals, the value found in the diabetic rats averaging 87.7 ± 6.2 % (*n* = 15) of that recorded, within the same experiments and at the same time, in the control rats (100.0 ± 6.2 %; *n* = 16).

In the kidney, the radioactive content, expressed relative to the paired plasma value, was significantly lower (*P* < 0.01) in STZ rats than in control animals, with a mean value of 68.1 ± 6.5 % (*n* = 11) of the corresponding readings made within the same experiments and at the same time in normal rats (i.e. 100.0 ± 8.7 %, *n* = 12).

Although the paired ratio in the radioactive content of each tissue and plasma was different in the two series of experiments listed in Table 1, the paired comparisons between muscle/liver, pancreas/liver and pancreas/muscle were virtually superimposable in these two sets of measurements (Table 4). Up to the 60^{th} min after D-[³H]mannoheptulose injection, the muscle/liver ratio did not exceed 17.00 ± 1.17 % (*n* = 24), and only became higher 48 hours after such an injection. A comparable time course was observed for the pancreas/liver ratio, which, up to the 60^{th} min after administration of the tritiated hexose, was higher (*P* < 0.02), however, than the corresponding muscle/liver ratio, it averaging 21.26 ± 1.07 % (*n* = 24). Such a difference was borne out by the finding that the paired pancreas/muscle ratio (125.71 ± 7.83 %; *n* = 24) was indeed higher (*P* < 0.005) than unity up to the 60^{th} min after D-[³H]mannoheptulose intravenous delivery.

The results collected in isolated pancreatic islets document the following features. First, even after extensive washing, the apparent distribution space of D-[³H]mannoheptulose, relative to the ³HOH distribution space, was at least one order of magnitude higher in isolated islets than in pieces of pancreatic tissue. Second, the uptake of D-[³H]mannoheptulose by isolated islets represented a time-related and temperature-sensitive process, inhibited by cytochalasin B and enhanced by D-glucose. Last, the apparent distribution space of the tritiated heptose (0.1 mM) was lower than that of D-[5-³H]glucose (8.3 mM). These findings were recently confirmed in experiments conducted in rat pancreatic islets separated from the incubation medium by centrifugation through an oil layer. The fact that D-glucose increased the radioactive content of the islets exposed to D-[³H]mannoheptulose might be due to stimulation by the hexose of the glucokinase-catalyzed phosphorylation of the tritiated heptose.

The measurements made in pieces of pancreatic tissue differed from those made in isolated islets in several respects. The radioactive content of pancreatic pieces was much lower than that of isolated islets, all data being collected under identical experimental conditions. The limited uptake of the tritiated heptose by pancreatic pieces represented a temperature-sensitive process in both control and STZ rats. D-glucose decreased the apparent distribution space of D-[³H]mannoheptulose in pancreatic pieces obtained from STZ, but not control, rats. Cytochalasin B apparently failed to affect significantly the radioactive content of pancreatic pieces exposed to D-[³H]mannoheptulose. Last, after repeated washes both before and after a second incubation of 60 min at 37°C in the presence of cytochalasin B, the distribution space of D-[³H]mannoheptulose greatly exceeded that of ³HOH.

At variance with unesterified D-[³H]mannoheptulose, its hexaacetate ester penetrated efficiently and in a time-related manner in acinar cells.

The obvious differences between D-[³H]mannoheptulose handling by islet *versus* acinar pancreatic cells show that the heptose (or a suitable radioactive analog) may be used to label preferentially the endocrine moiety of the pancreatic gland. The apparent distribution space of D-[³H]mannoheptulose by pancreatic pieces was lower in STZ rats than in control animals. Although this difference was most obvious after a short period (10 min) of exposure to the tritiated heptose, it could still be detected after 60 min incubation, when the pieces were further incubated for 60 min in the nominal absence of D-[³H]mannoheptulose but presence of cytochalasin B. Likewise, in experiments conducted *in vivo,* the radioactive content of the pancreas was significantly lower in STZ rats than in control animals, whether shortly (15 min) or much later (48 hours) after intravenous administration of D-[³H]mannoheptulose.

The postulated participation of GLUT2 in the transport of D-[³H]mannoheptulose across the plasma membrane is further supported by the data listed in Table 4, indicating that, at least up to the 60^{th} min after intravenous injection of the tritiated heptose, the radioactive content was much higher in liver than pancreas, and slightly but significantly higher in the pancreatic gland than in muscle.

### Example 4: Uptake of D-mannoheptulose by Normal and Tumoral Pancreatic Islet Cells

D-[³H]mannoheptulose uptake by rat pancreatic islets or dispersed islet cells was found to represent a time-related and temperature-sensitive process inhibited by cytochalasin B. This mould metabolite also inhibited the efflux of D-[³H]mannoheptulose from prelabelled islets. After 60 min incubation at 37°C, the apparent intracellular distribution space of the tritiated heptose was close to or somewhat higher than that of D-[5-³H]glucose and close to 50 % of the intracellular ³HOH space. It was further enhanced by D-glucose and a high concentration of 10 mM of D-mannoheptulose. The uptake of D-[³H]mannoheptulose was much lower however than that of D-[³H]mannoheptulose hexaacetate. As judged from the fate of D-mannoheptulose hexa[2-¹⁴C]acetate, the latter ester was efficiently hydrolysed in the islet cells. The internalisation of D-[³H]mannoheptulose (or its ester) coincided with the generation of tritiated acidic metabolites, reflecting phosphorylation of the heptose. The situation found in normal islet cells sharply differed from that found in tumoral islet cells of either the RINm5F or INS-1 line, in which the apparent distribution space of D-³H]mannoheptulose represented only about 3 and 9 %, respectively, of the intracellular ³HOH space. These results indicate that the entry of D-mannoheptulose into islet cells represents a carrier-mediated process, possibly mediated at the intervention of GLUT2.

The results collected in rat isolated islets indicate that [U-¹⁴C]sucrose, rather than L-[1-¹⁴C]glucose, represents a suitable tool for measurement of the extracellular space.

The effect of D-glucose and, in high concentration, of D-mannoheptulose upon the intracellular distribution space of D-[³H]mannoheptulose thus appears to be rather due to a change in the balance between the influx and efflux of the tritiated heptose across the plasma membrane.

In pancreatic islets, the apparent distribution space of D-[³H]mannoheptulose hexaacetate (0.1 mM) largely exceeded that of unesterified D-[³H]mannoheptulose (also 0.1 mM). The measurement of acidic metabolites in islets exposed to D-mannoheptulose hexa[2-¹⁴C]acetate indicated that the ester underwent extensive hydrolysis in the islet cells.

A vastly different situation was found in tumoral islet cells of the RINm5F or INS-1 cell lines, in which the distribution space of D-[³H]mannoheptulose was as low as in erythrocytes or parotid cells. In fair agreement with such findings, these two lines of tumoral cells were resistant to D-mannoheptulose in terms of the effect of the heptose upon D-glucose catabolism. The sole significant difference detected between these two types of tumoral cells consisted in a greater uptake of D-[³H]mannoheptulose in INS-1 cells than in RINm5F cells.

### Example 5: Uptake of Tritiated D-Mannoheptulose by Liver, Pancreatic Exocrine and Endocrine Cells

Female inbred C57BL/6 or BALB/c mice (Iffa Credo, L'Arbresle, France) given free access to food and water were injected i.p. with streptozotocin (1.0 µmol/g body wt.; Sigma, Bornem, Belgium) dissolved in a citrate buffer (pH 6.4). Five to 7 days later, mice with two consecutive daily glycemia in excess of 16.7 mM (Accutrend Sensor blood glucose monitor; Boehringer Mannheim, Mannheim, Germany) were transplanted under the left renal capsule with groups of 300-550 islets each obtained by the collagenase procedure from animals of the same strain. Seven to 14 days after islet transplantation, [U-¹⁴C]sucrose (5 µCi/mouse) and D-[5-³H]mannoheptulose (10-100 µCi/mouse) both obtained from NEN Life Science Products (Zaventem, Belgium) and placed in a saline solution (0.1 ml/rat) also containing 0.1 mM of unlabelled sucrose and D-mannoheptulose, was injected in a tail vein. One hour later, the animals were killed by decapitation and blood collected in heparinized tubes for measuring plasma radioactivity.

Liver pieces (119.2 ± 18.1 mg wet wt.; *n* = 10; mean ± SEM in this and all further instances), pancreatic glands (173.8 ± 14.9 mg wet wt.; *n* = 10), and fragments of kidney capsule with the transplanted islets were then removed and also examined for their radioactive content. The DNA content of the pancreas averaged 3.21 ± 0.20 µg/mg wet wt. (*n* = 10). The DNA content of the islet graft averaged 12.2 ± 1.7 µg (n = 10). It exceeded by 7.8 ± 1.3 µg (*n* = 6; *P* < 0.005) the DNA content of the contralateral kidney capsule sample. Assuming a mean content of 7 pg DNA per somatic, non-dividing cell and a mean number of 3,000 cells per islet, such a difference would correspond to about 370 ± 60 islets/sample, as compared to a nominal number of 435 ± 37 transplanted islets.

All results, including those already mentioned are presented as mean values (± SEM), together with the number of individual determinations (*n*). The statistical significance of differences between mean values was assessed by use of Student's *t*-test.

Tritiated D-mannoheptulose, a ketoheptose known to inhibit D-glucose metabolism in hepatocytes and pancreatic islets, but not so in pancreatic acinar cells, was injected intravenously in streptozotocin-induced diabetic mice transplanted under the kidney capsule with islets from control mice of the same strain. One hour after the injection of the tritiated heptose, the radioactive content was 5-8 times higher in the liver and transplanted islets than in the pancreatic gland.

The present results indicate that the uptake of tritiated D-mannoheptulose is higher in hepatocytes or islet cells than in acinar pancreatic cells. D-[³H]mannoheptulose could thus conceivably be used to assess, in the isolated perfused pancreas, the total islet mass in animal models of type-1 or type-2 diabetes by comparison of results obtained in untreated and STZ-injected animals. The present findings may also pave the way to the use of a suitably labelled D-mannoheptulose analog (e.g. ¹¹C-labelled D-mannoheptulose or 7-deoxy-7-[¹²³I]iodo-D-mannoheptulose) for the non-invasive quantification of the endocrine pancreas, e.g. in human subjects.

## Claims

1. Labelled D-mannoheptulose or labelled D-mannoheptulose derivatives used for imaging endocrine pancreas tissues or cells.

2. Labelled D-mannoheptulose or labelled D-mannoheptulose derivatives according to claim 1, which are radioactive D-mannoheptulose or radioactive D-mannoheptulose derivatives comprising a radioactive atom or labelled with a radioactive molecule.

3. Labelled D-mannoheptulose or labelled D-mannoheptulose derivatives according to claim 2, wherein the radioactive atom is radioactive carbon, fluor or iode or a molecule comprising radioactive carbonic fluor or iode, preferably selecting from the group consisting of ¹¹C, ¹⁸ F, ¹²³I and/or ¹³¹I.

4. Labelled D-mannoheptulose or labelled D-mannoheptulose derivatives according to any of the claims 1 to 3, which are selected from the group consisting of 2-deoxy-2-iodo-D-mannoheptulose, 6-deoxy-6-iodo-D-mannoheptulose, 7-deoxy-7-iodo-D-mannoheptulose, 1-deoxy-1-iodo-D-mannoheptulose and their iodophenyl analogues.

5. A kit comprising the labelled D-mannoheptulose or labelled D-mannoheptulose derivatives according to any of the preceding claims 1 to 4.

6. A method for imaging endocrine pancreas tissues or cells of a patient comprising the step of administrating to said patient of a suitable amount of labelled D-mannoheptulose or labelled D-mannoheptulose derivatives according to any of the preceding claims 1 to 4 detecting and possibly recording a signal obtained from said labelled D-mannoheptulose or labelled D-mannoheptulose derivatives in the endocrine pancreas tissues or cells of said patient by imaging detection (and recording) means.

7. The method according to the claim 6, further comprising the step of correlating the signal obtained to a corresponding reference signal obtained from a healthy patient.

8. A computer program comprising program code means for performing the step according to any of the preceding claims 6 or 7, when said program is run on a computer.

9. A computer program product comprising program code means stored on a computer's readable means for performing the step according to the method of claim 6 or 7, when said program is run on a computer.
